# EUROPEAN PATENT APPLICATION

(11) **EP 2 338 865 A1**
(43) Date of publication of application: **29.06.2011**
(21) Application number: 09180443.5
(22) Date of filing: 22.12.2009
(51) Int. Cl.: C07C 7/00, C07C 7/11, C07C 11/02

(54) **Process for removing oxygenated contaminants from an hydrocarbon stream**

(71) Applicant: Total Petrochemicals Research Feluy, 7181 Seneffe (Feluy) (BE)
(72) Inventor: Avaullee, Laurent, 6183 Trazegnies (BE); Thoret Bauchet, Jean-Pierre, 1180 Bruxelles (BE)

(57) **Abstract**

The present invention is a process for removing oxygenated contaminants and water from an hydrocarbon stream comprising :
introducing the contaminated hydrocarbon stream in a gaseous phase in an absorption zone,
contacting said hydrocarbon stream in said absorption zone with an absorbent capable to absorb water and oxygenated contaminants at conditions effective to produce,
● an overhead hydrocarbon stream having a reduced oxygenated contaminants and water content and
● an absorbent bottoms stream comprising the absorbent, hydrocarbons and having an enhanced oxygenated contaminants and water content, introducing the above absorbent bottoms stream in a stripping zone at conditions effective to produce,
● an absorbent bottoms stream essentially free of hydrocarbons, oxygenated contaminants and water and
● an overhead stream comprising essentially hydrocarbons, water and the oxygenated contaminants,

recycling the absorbent bottoms stream of the stripping zone to the absorption zone,
optionally fractionating the overhead stream from the stripping zone to recover the hydrocarbons,
sending the overhead of the absorption zone to a caustic wash to remove the acidic components and recovering an hydrocarbon stream essentially free of water and oxygenated contaminants.

## Description

### [Field of the invention]

The present invention is a process for removing oxygenated contaminants from an hydrocarbon stream. In a specific embodiment said hydrocarbon stream comprises olefins.

Olefins are traditionally produced from petroleum feedstocks by catalytic or steam cracking processes. These cracking processes, especially steam cracking, produce light olefin(s), such as ethylene and/or propylene, from a variety of hydrocarbon feedstock. Ethylene and propylene are important commodity petrochemicals useful in a variety of processes for making plastics and other chemical compounds.

The limited supply and increasing cost of crude oil has prompted the search for alternative processes for producing hydrocarbon products. The MTO process produces light olefins such as ethylene and propylene as well as heavy hydrocarbons such as butenes. Said MTO process is the conversion of methanol or dimethylether by contact with a molecular sieve. The interest in the methanol to olefin (MTO) process is based on the fact that methanol can be obtained from coal or natural gas by the production of synthesis gas which is then processed to produce methanol.

The effluent produced by a MTO process is a complex mixture comprising the desired light olefins, unconverted oxygenates, by-product oxygenates, heavier hydrocarbons and large amounts of water. The separation and purification of this mixture to recover the light olefins and other valuable byproducts is critical to the overall efficiency and cost effectiveness of the process. In particular, it is important that the purification scheme produces products that are substantially free of impurities, which could adversely effect downstream processing. For example, certain oxygenate components present in the effluent from an oxygenate conversion process, particularly aldehydes and ketones, may cause problems in olefin recovery operations and in derivative manufacturing processes that feed and react C₄+ hydrocarbons. There is therefore a need to ensure that the effluent purification scheme effectively removes aldehydes and ketones from the olefinic and C₄+ hydrocarbon components while at the same time minimizing loss of useful product.

Olefins can also be produced by dehydration of the corresponding alcohol. Ethanol can be obtained by fermentation of carbohydrates. Made up of organic matter from living organisms, biomass is the world's leading renewable energy source. The effluent produced by the ethanol dehydration comprises essentially unconverted ethanol, water, ethylene, acetaldehyde. Acetaldehyde may cause problems in ethylene recovery operations.

### [Background of the invention]

WO 2006-048098 A1 describes a method for removing oxygen-containing organic compounds from mixtures of various hydrocarbon compounds. A liquid phase containing hydrocarbons and oxygenates is supplied to a first column to produce a light fraction comprising the oxygenates and a bottom fraction. Said light fraction and a gaseous mixture of hydrocarbons and oxygenates are then supplied to a second column. A separation by distillation into a light and a heavy hydrocarbon fraction takes place in said second column, wherein an additional solvent is fed to the upper part of said second column which dissolves the oxygenates prior to discharging them in the bottom product of said second column. As a result, an oxygenate-free hydrocarbon product exits the head of the second column and a mixture of oxygenates, solvents and remaining hydrocarbons is removed from the bottom of the second column. The solvent can be partially or completely regenerated and recycled to the extractive distillation column. The solvent can be an alcohol such as methanol, ethanol, propanol or diethyleneglycol or N-MethylPyrrolidone (NMP). The examples are made with methanol and NMP.

US 20030045655 A1 provides a method of extracting an oxygenate from an olefin containing stream. The method comprises contacting the olefin containing stream with an extractant; and separating the contacted olefin containing stream and extractant using extractive distillation. Preferably, the extractant is a polar liquid composition at 1 atm., having an average boiling point of at least 38° C at 1 atm. More preferably, the polar liquid composition comprises at least 75 wt. % water, alcohol, or a mixture thereof. The extractants are also desirably polar compositions. Such compositions preferably contain compounds such as water, monohydric alcohols, polyhydric alcohols, or mixtures thereof. Preferred monohydric alcohols include ethanol and propanol. Preferred polyhydric alcohols include glycols. Preferred glycols include ethylene glycol and triethylene glycol. It is desirable that the extractant contains at least about 75 wt. % water, monohydric alcohol, and or polyhydric alcohol, preferably at least about 85 wt. %, more preferably at least about 90 wt. %, and most preferably at least about 95 wt. %. Water is most preferred as the extractant.

US 20030098281 A1 describes a method of controlling water and/or oxygenate concentrations of an olefin stream. The method includes contacting the olefin stream with a liquid absorbent. The liquid absorbent is selected from the group consisting of a polyol, amine, amide, nitrile, heterocyclic nitrogen containing compound, and mixtures thereof.

WO 03 020678 A2 describes a method of removing dimethyl ether from an olefin stream made from an oxygenate to olefin reaction process, comprising: contacting oxygenate with a molecular sieve catalyst to form an olefin stream, wherein the olefin stream comprises ethylene, propylene, dimethyl ether and C4+ olefin and higher boiling point hydrocarbons, separating the olefin stream into a first stream comprising the ethylene, propylene and dimethyl ether and a second stream comprising the C4+ olefin and higher boiling point hydrocarbons, and separating the dimethyl ether present in the first stream using extractive distillation. The extractants are desirably polar compositions. Such compositions preferably contain compounds such as water, monohydric alcohols, polyhydric alcohols, or mixtures thereof. Preferred monohydric alcohols include ethanol and propanol. Preferred polyhydric alcohols include glycols. Preferred glycols include ethylene glycol and triethylene glycol. It is desirable that the extractant contains at least about 75 wt.% water, monobydric alcohol, and or polyhydric alcohol, preferably at least about 85 wt.%, more preferably at least about 90 wt.%, and most preferably at least about 95 wt.%. Water is most preferred as the extractant.

WO 03 020670 A1 provides a method for removing oxygenated components such as acetaldehyde, CO2 and/or water from an olefin stream. It explains it is desirable to remove such oxygenated components, since they may poison catalysts that are used to further process olefin composition. In addition, the presence of certain oxygenated compounds, such as acetaldehyde, can cause fouling in other olefin purification units, e.g., acid gas treating units. This prior art provides a method of treating an ethylene and/or propylene containing stream. The method comprises providing an olefin stream containing ethylene, propylene, C4+ olefins and acetaldehyde. The olefin stream is separated into a first fraction and a second fraction, wherein the first fraction comprises at least a majority of the ethylene and/or propylene present in the olefin stream, and the second fraction comprises at least a majority of the C4+ olefins and acetaldehyde present in the olefin stream. The first fraction is then acid gas treated. The olefin stream is separated by distillation, preferably, the distillation is extractive distillation using an extractant. The preferred extractant is a polar composition having an average boiling point of at least 38°C at 1 atm. Methanol is one type of preferred extractant.

WO 03 020672 A1 describes method of removing dimethyl ether from an ethylene and/or propylene containing stream. The olefin stream is passed to a water absorption column, methanol is used as the water absorbent. Methanol and entrained water, as well as some oxygenated hydrocarbon, is recovered as the bottoms stream of said water absorption column, an overhead olefin is recovered and sent to a distillation column. The distillation column separates ethylene and propylene, as well as lighter boiling point components from the dimethyl ether and heavier boiling point components, including C4 + components and methanol remaining from the methanol wash. Additional methanol is added to the distillation column to reduce clathrate and/or free water formation in the distillation column. The ethylene and propylene containing stream exits the distillation column as overhead and the heavier boiling point components which include the dimethyl ether and C4 + components exit the distillation column as the bottoms. Ethylene and propylene then flow to a caustic wash column.

WO 03 033438 A1 describes a method for processing an olefin stream containing oxygenates and water, comprising: providing an olefin stream containing oxygenates and water; dewatering the olefin stream; compressing the dewatered olefin stream; washing the olefin stream with methanol to remove at least a portion of the oxygenate from the olefin stream; contacting the methanol washed olefin stream with water; and fractionating the water contacted olefin stream. The olefin stream is the effluent of an MTO process.

US 2006 258894 A1 relates to a process for extracting oxygenates from a hydrocarbon stream, typically a fraction of the condensation product of a Fischer-Tropsch reaction, while preserving the olefin content of the condensation product. The oxygenate extraction process is a liquid-liquid extraction process that takes place in an extraction column using a polar organic solvent, such as methanol, and water as the solvent, wherein the polar organic solvent and water are added separately to the extraction column.

US 2009 048474 A1 relates to a process for the production of alkene(s) from a feedstock comprising at least one monohydric aliphatic paraffinic primary (or secondary) alcohol(s), consisting of ethanol or propanol(s) or a mixture thereof, characterised by the following steps;
1. the monohydric aliphatic paraffinic primary (or secondary) alcohol(s) are converted into the corresponding same carbon number alkene(s) in a reactive distillation column at elevated pressure and temperature so that the heads stream extracted from the top of the said reactive distillation column comprises essentially the said alkene(s),
2. the heads stream from step 1 is then cooled to a temperature sufficient to condense at least part of the alkene(s) with the highest boiling point,
3. at least part of the condensed alkene(s) from step 2 are then recycled back into the said reactive distillation column, as a reflux return,
4. simultaneously the remaining alkene(s) are recovered.

### [Brief summary of the invention]

The present invention is a process for removing oxygenated contaminants and water from an hydrocarbon stream comprising :
introducing the contaminated hydrocarbon stream in a gaseous phase in an absorption zone,
contacting said hydrocarbon stream in said absorption zone with an absorbent capable to absorb water and oxygenated contaminants at conditions effective to produce,
   ● an overhead hydrocarbon stream having a reduced oxygenated contaminants and water content and
   ● an absorbent bottoms stream comprising the absorbent, hydrocarbons and having an enhanced oxygenated contaminants and water content, introducing the above absorbent bottoms stream in a stripping zone at conditions effective to produce,
   ● an absorbent bottoms stream essentially free of hydrocarbons, oxygenated contaminants and water and
   ● an overhead stream comprising essentially hydrocarbons, water and the oxygenated contaminants,
recycling the absorbent bottoms stream of the stripping zone to the absorption zone,
optionally fractionating the overhead stream from the stripping zone to recover the hydrocarbons,
sending the overhead of the absorption zone to a caustic wash to remove the acidic components and recovering an hydrocarbon stream essentially free of water and oxygenated contaminants.

The hydrocarbon stream comprising oxygenated contaminants and water can be a stream in a refinery or a chemical plant. The hydrocarbon may comprise olefins.

In an embodiment the hydrocarbon stream comprising oxygenated contaminants and water is the effluent produced by an alcohol dehydration to make at least an olefin. By way of example the effluent produced by the ethanol dehydration comprises essentially unconverted ethanol, water, ethylene, acetaldehyde.

In another embodiment the hydrocarbon stream comprising oxygenated contaminants and water is the effluent produced by a MTO process. Said hydrocarbon stream is a complex mixture comprising the desired light olefins, unconverted oxygenates, by-product oxygenates, heavier hydrocarbons and large amounts of water.

### [Detailed description of the invention]

As regards the oxygenated contaminants one can cite alcohols such as methanol, ethanol, C3 alcohols; ethers such as dimethyl ether, diethylether and methyl ethyl ether; carboxylic acids such as acetic acid, propanoic acid and butyric acid; aldehydes such as acetaldehyde; ketones such as acetone; and esters such as methyl esters. Particularly problematic oxygenate contaminants in an alcohol dehydration are aldehydes. Particularly problematic oxygenate contaminants in the MTO process are dimethyl ether (DME) and acetaldehyde.

The hydrocarbon stream comprising oxygenated contaminants and water can be available at low pressure such as 1 to 3 bars absolute and may comprise a high proportion of water. Advantageously said hydrocarbon stream is successively compressed and cooled in one or more steps to remove the major part of water and further fed to the absorption zone. The pressure of the absorption zone is advantageously ranging from 5 to 40 bars absolute and preferably from 10 to 30 bars absolute.

In the previous compression steps the recovered water contains a part of the oxygenated contaminants and hydrocarbons dissolved. In an embodiment the water recovered upon each cooling further to a compression step is sent to a stripping column referred to as the water stripping column to produce an overhead stream comprising essentially oxygenated contaminants and hydrocarbons and an essentially pure water bottoms stream. Optionally the overhead stream is burned to destroy the oxygenated contaminants and recover heat. The contaminated hydrocarbon stream can also be cooled before the first compression step and water recovered.

In a specific embodiment the recycled absorbent to the absorption zone is cooled before entering into said absorption zone. Advantageously it is cooled to about 30°C or lower, preferably to about 20°C or lower.

The proportion of the oxygenated contaminants in the hydrocarbon stream comprising oxygenated contaminants and water can be up to 5 w%.

Advantageously the absorbent is selected from the group consisting of a polyol, amine, amide, nitrile, heterocyclic nitrogen containing compound, and mixtures thereof. Examples of polyol, amine, amide, nitrile, heterocyclic nitrogen containing compounds which can be used include ethylene glycol, diethylene glycol, triethylene glycol, ethanolamine, diethanolamine, triethylamine, hindered cyclic amines, acetonitrile, n-methylpyrrolidone, and dimethyl formamide, as well as mixtures of any two or more of these compounds. Olefins treated in accordance with this invention are particularly suitable for use as feedstock for making polyolefins.

Substantial amounts of water and oxygenated contaminants are removed from the hydrocarbon vapor stream by contacting the vapor stream with an effective amount of absorbent. It is preferred that the absorbent be a polyol, amine, amide, nitrile, and/or heterocyclic nitrogen containing compound. This type of absorbent is particularly desirable, since it will remove such hard to remove contaminants as dimethylether, acetaldehyde and water, yet it will not readily absorb olefins optionally present in the hydrocarbon stream. This means that oxygenated contaminants can be removed from an olefin stream with a very high efficiency.

To obtain a high degree of effectiveness, the absorbent material introduced into the absorption system should have little non-oxygenated hydrocarbon absorbing material, such as a diluent. For example, the absorbent material introduced into an absorber should contain at least about 75 wt % absorbent material that is effective in removing dimethylether and/or water from an olefin stream rich in ethylene and/or propylene. Desirably, the absorbent material should contain at least about 90 wt %, preferably at least about 95 wt %, more preferably at least about 98 wt % absorbent. Examples of absorbents include at least one compound selected from the group consisting of ethylene glycol, diethylene glycol, triethylene glycol, ethanolamine, diethanolamine, triethylamine, hindered cyclic amines, acetonitrile, n-methylpyrrolidone, dimethyl formamide, and combinations thereof.

Conventional absorption systems can be used in this invention. In one embodiment, the absorption system uses packed columns, although plate absorption columns may also be used. In another embodiment, the absorption column has a liquid inlet located at a top portion of the absorption column. The absorbent liquid is evenly distributed across the top of the column. Desirably, an even distribution of the absorbent liquid is accomplished by using a distributor plate or spray nozzles. At the bottom of the absorption column is a gas inlet where thehydrocarbon stream, containing water and oxygenated contaminants, enters the absorption column. The vapor components move up the column countercurrent to the liquid absorbent moving down the column. This is known as countercurrent absorption. The packing or plates in the column provides a surface for intimate contact between the vapor and liquid components within the column. In a countercurrent absorption column, the concentration of soluble gasses in both the liquid and vapor phases is greatest at the bottom of the column, and lowest at the top of the column. The outlet for the liquid is at the bottom of the absorption column, typically below the gas inlet. The outlet for the gas phase lean in the gasses most soluble in the liquid absorbent is at the top of the absorption column, typically above the liquid inlet.

In a specific embodiment the stripping zone is a distillation column. The overhead stream of said distillation column of the stripping zone is cooled to produce an aqueous phase comprising oxygenated contaminants and a gaseous phase comprising hydrocarbons and oxygenated contaminants. A part of said aqueous phase is used as the reflux of the distillation column, the remaining part is optionally sent to the above cited water stripping column.

Advantageously the above gaseous phase is sent to a wash column fed with water to produce an overhead hydrocarbon stream comprising oxygenated contaminants and a bottoms aqueous stream comprising oxygenated contaminants. Advantageously the said bottoms of the wash column and the remaining part of the aqueous phase recovered by cooling the stripping column overhead in the stripping zone and not used as a reflux comprise an essential portion of the oxygenated contaminants to be removed. More precisely in case the contaminated hydrocarbon stream is compressed before entering the absorption zone a part of the oxygenated contaminants goes in the condensed water. The sum of,
the oxygenated contaminants in the said condensed water,
the oxygenated contaminants in the bottoms of the wash column,
the remaining part of the aqueous phase recovered by cooling the stripping column overhead in the stripping zone and not used as a reflux, comprise the essential portion of the oxygenated contaminants to be removed, advantageously more than 90w%. Advantageously the overhead stream of the wash column comprises a very small portion of the oxygenated contaminants to be removed.

Optionally the said overhead of the wash column is recycled to the process which has produced the hydrocarbon stream comprising oxygenated contaminants and water to be purified. Optionally the aqueous bottoms stream is sent to the above cited water stripping column.

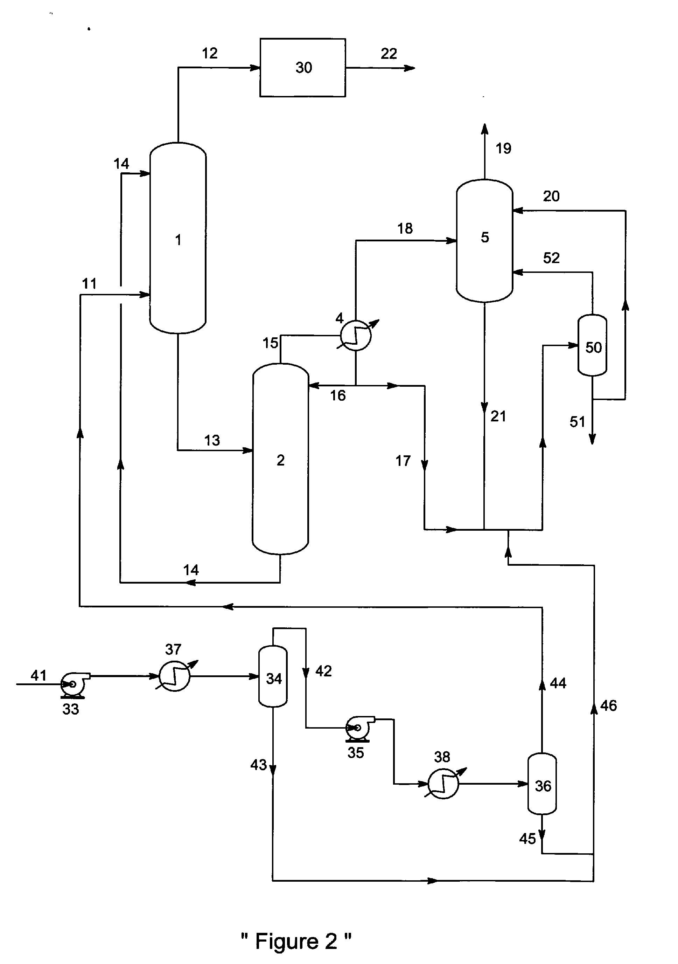

In an embodiment,
● the water stream recovered in the course of the compression of the contaminated hydrocarbon before entering the absorption zone,
● the bottoms of the wash column,
● the remaining part of the aqueous phase recovered by cooling the stripping column overhead in the stripping zone and not used as a reflux, are sent to a flash drum to produce a gaseous overhead sent to the wash column, advantageously in the lower part, to be washed and an aqueous bottoms stream sent in part to the wash column and used as absorbent. The remaining part of said aqueous bottoms stream contains the major part of the oxygenated contaminants to be removed. This embodiment is illustrated in fig 2.

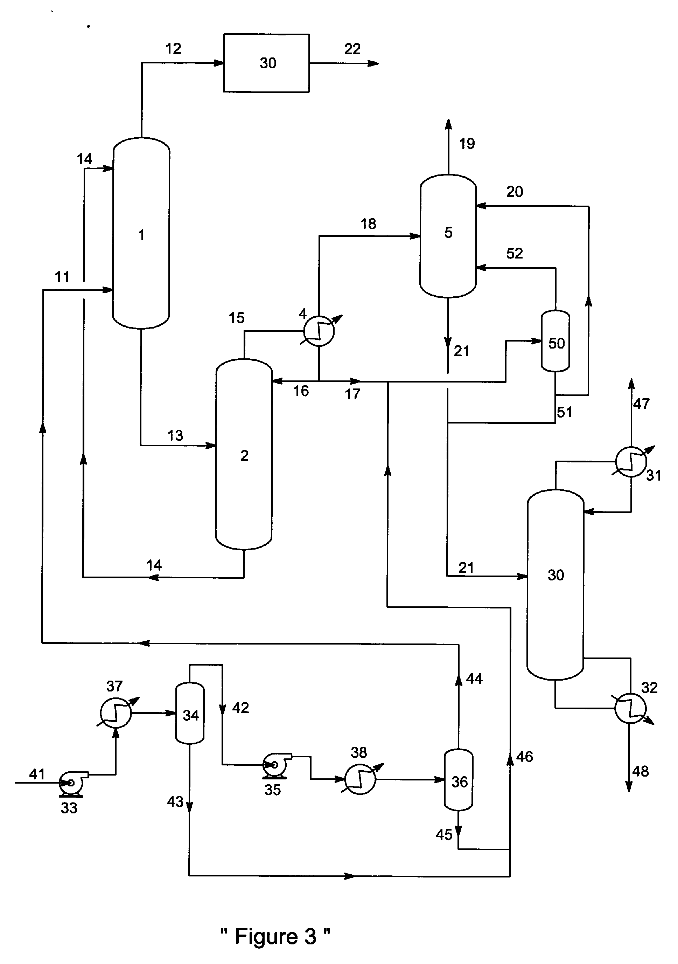

In an embodiment,
● the water stream recovered in the course of the compression of the contaminated hydrocarbon before entering the absorption zone,
● the remaining part of the aqueous phase recovered by cooling the stripping column overhead in the stripping zone and not used as a reflux, are sent to a flash drum to produce a gaseous overhead sent to the wash column, advantageously in the lower part, to be washed and an aqueous bottoms stream sent in part to the wash column and used as absorbent. The remaining part of said flash drum aqueous bottoms stream and the bottoms of the wash column are sent to a stripping column referred to as the water stripping column to produce an overhead stream comprising essentially oxygenated contaminants and hydrocarbons and an essentially pure water bottoms stream. Optionally the overhead stream is burned to destroy the oxygenated contaminants and recover heat. Said overhead stream contains the major part of the oxygenated contaminants to be removed. This embodiment is illustrated in fig 3.

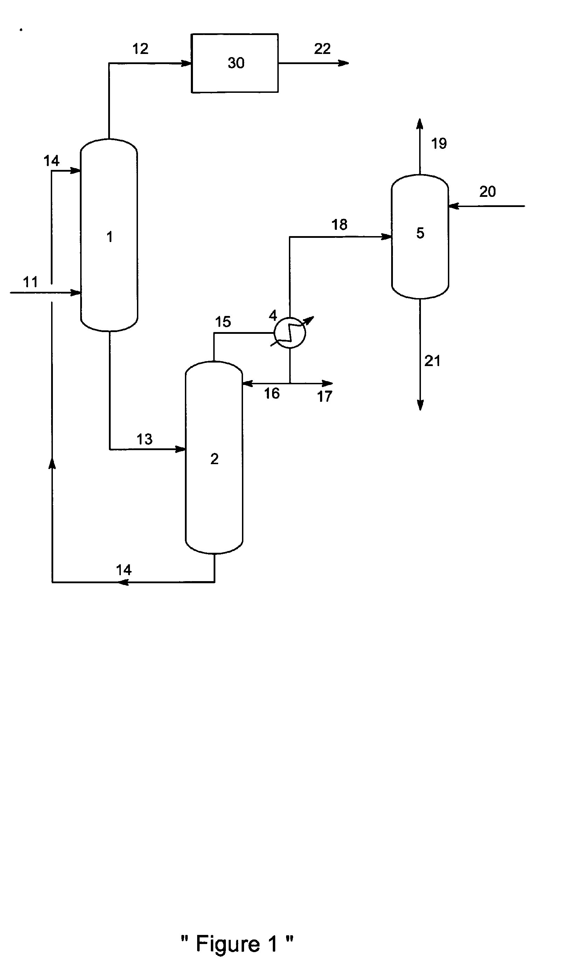

Fig 1 depicts an embodiment of the invention. 1 is the absorption zone, 2 the stripping zone, 3 the caustic wash, 4 a condenser-separator and 5 a wash column. The hydrocarbon stream 11 comprising oxygenated contaminants and water is fed to the absorption zone 1 to produce an overhead hydrocarbon stream 12 having a reduced oxygenated contaminants and water content and an absorbent bottoms stream 13 comprising the absorbent, hydrocarbons and having an enhanced oxygenated contaminants and water content. The absorbent bottoms stream 13 is sent in a stripping zone 2 to produce an absorbent bottoms stream 14 essentially free of hydrocarbons, oxygenated contaminants and water and an overhead stream 15 comprising essentially hydrocarbons, water and the oxygenated contaminants. The reboiler at bottoms of column 2 is not displayed on this fig 1. The stream 14 is cooled and recycled to the absorption zone 1. The stream 15 is condensed in a condenser separator 4 to produce a reflux 16, an aqueous phase 17 comprising oxygenated contaminants and a gaseous phase 18 comprising essentially hydrocarbons and oxygenated contaminants. The stream 18 is sent to a wash column 5 fed with water 20 to produce an overhead hydrocarbon stream 19 comprising hydrocarbons and a small portion (advantageously less than about 10w%) of the oxygenated contaminants of the stream 11 and a bottoms aqueous stream 21 comprising oxygenated contaminants. The streams 17 and 21 comprise the essential portion (advantageously more than about 90w%) of the oxygenated contaminants of stream 11. Optionally the overhead 19 is recycled to the process which has produced the hydrocarbon stream comprising oxygenated contaminants and water. The overhead 12 of the absorption zone is sent to a caustic wash 3 to remove the acidic components and recovering a hydrocarbon stream 22 essentially free of water, acidic components and oxygenated contaminants.
Fig 2 depicts an embodiment of the invention and derives from fig 1 by incorporation of the flash drum 50; compressors 33, 35; separators 34, 36; coolers 37,38. Streams 17 and 21 are sent to flash drum 50. The hydrocarbon stream 41 comprising oxygenated contaminants and water is sent to a compressor 33, cooled in a heat exchanger 37 and sent to a separator 34 to produce a gaseous phase 42 and an aqueous phase 43. The gaseous phase 42 is sent to a compressor 35, cooled in a heat exchanger 38 and sent to a separator 36 to produce a gaseous phase 44 and an aqueous phase 45. The gaseous stream 44 which is similar to the stream 41, but has a reduced water and oxygenated contaminants content, is sent via line 11 to the absorption zone. The aqueous streams 43 and 45 which are essentially water containing a part of the oxygenated contaminants and hydrocarbons dissolved are sent to the flash drum 50. The gaseous stream 52 from the flash drum 50 is sent to wash column 5. Aqueous stream 51 from flash drum 50 contains oxygenated contaminants. A part of the flash drum bottoms is sent as stream 20 to the wash column 5 and used as absorbent.
Fig 3 depicts an embodiment of the invention and derives from fig 2 by incorporation of water stripping column 30 comprising a condenser 31 and a reboiler 32. Aqueous streams 51 from flash drum 50 and stream 21 from wash column are sent to stripping column 30. The water stripping column 30 produces an overhead stream 47 comprising essentially hydrocarbons and oxygenated contaminants and an essentially pure water bottoms stream 48. Stream 47 could be destroyed or recycled.

As regards alcohol dehydration, such process is described in WO-2009-098262, WO-2009-098267, WO-2009-098268 and WO-2009-098269 the content of which is incorporated in the present application. The present invention is very efficient for the purification of ethylene produced by dehydration of ethanol.

Typical weight composition of the effluent to be purified further to the ethanol dehydration are on a dry basis, the total being 100% :

| | | | |
|---|---|---|---|
| CARBON MONOXIDE | 0.01 | to | 0.1 |
| ETHANE | 0.01 | to | 0.1 |
| ETHYLENE | 95 | to | 99.75 |
| PROPYLENE | 0.0 | to | 0.01 |
| ACETALDEHYDE | 0.03 | to | 0.3 |
| ETHANOL | 0.2 | to | 2.0 |
| ISOBUTYLENE | 0.0 | to | 0.1 |
| 1-BUTENE | 0.0 | to | 0.1 |
| TRANS-2-BUTENE | 0.0 | to | 0.3 |
| CIS-2-BUTENE | 0.0 | to | 0.3 |
| 3-METHYL-1-BUTENE | 0.0 | to | 0.3 |

The proportion of water may be from 1 mole of water for one mole of ethylene to about five tons for one ton of ethylene. The above typical stream above can be purified with the process of the invention to get an ethylene stream having an acetaldehyde content of less than 5 ppm, often less than 3 ppm more often less than 2 ppm.

As regards the MTO process, such process is described in WO-2008-110526, WO-2008-110528, WO-2008-110530, WO-2009-016153, WO-2009-016154, WO-2009-016155, WO-2009-092779, WO-2009-092780, WO-2009-092781, the content of which is incorporated in the present application. The MTO process has also been described in US 2006 0235251, WO 2005 016856, US 2006 0063956, US 2006 0161035, US 6207872, US 2005 0096214, US 6953767 and US 7067095, the content of which is incorporated in the present application.

The effluent produced by a MTO process which is an hydrocarbon stream comprising oxygenated contaminants and water can be purified with the process of the invention to get an ethylene/propylene stream having an acetaldehyde content of less than 5 ppm, often less than 3 ppm more often less than 2 ppm. Said stream can be purified with the process of the invention to get an ethylene/propylene stream having a DME content of less than 5 ppm, often less than 3 ppm more often less than 2 ppm.

## Claims

1. Process for removing oxygenated contaminants and water from an hydrocarbon stream comprising :
introducing the contaminated hydrocarbon stream in a gaseous phase in an absorption zone,
contacting said hydrocarbon stream in said absorption zone with an absorbent capable to absorb water and oxygenated contaminants at conditions effective to produce
● an overhead hydrocarbon stream having a reduced oxygenated contaminants and water content and
● an absorbent bottoms stream comprising the absorbent, hydrocarbons and having an enhanced oxygenated contaminants and water content, introducing the above absorbent bottoms stream in a stripping zone at conditions effective to produce
● an absorbent bottoms stream essentially free of hydrocarbons, oxygenated contaminants and water and
● an overhead stream comprising essentially hydrocarbons, water and the oxygenated contaminants,
recycling the absorbent bottoms stream of the stripping zone to the absorption zone,
optionally fractionating the overhead stream from the stripping zone to recover the hydrocarbons,
sending the overhead of the absorption zone to a caustic wash to remove the acidic components and recovering an hydrocarbon stream essentially free of water and oxygenated contaminants.

2. Process according to claim 1 wherein the hydrocarbon stream comprising oxygenated contaminants and water is successively compressed and cooled in one or more steps to remove the major part of water and further fed to the absorption zone.

3. Process according to any one of the preceding claims wherein the stripping zone is a distillation column.

4. Process according to claim 3 wherein the overhead stream of said distillation column of the stripping zone is cooled to produce an aqueous phase comprising oxygenated contaminants and a gaseous phase comprising hydrocarbons and oxygenated contaminants, a part of said aqueous phase is used as the reflux of the distillation column.

5. Process according to claim 4 wherein the gaseous phase produced by the cooling and comprising hydrocarbons and oxygenated contaminants is sent to a wash column fed with water to produce an overhead hydrocarbon stream comprising oxygenated contaminants and a bottoms aqueous stream comprising oxygenated contaminants.

6. Process according to claim 5 wherein the said overhead of the wash column is recycled to the process which has produced the hydrocarbon stream comprising oxygenated contaminants and water to be purified.

7. Process according to claims 5 and 2 or 6 and 2 wherein,
● the water stream recovered in the course of the compression of the contaminated hydrocarbon before entering the absorption zone,
● the bottoms of the wash column,
● the remaining part of the aqueous phase recovered by cooling the stripping column overhead in the stripping zone and not used as a reflux, are sent to a flash drum to produce a gaseous overhead sent to the wash column to be washed and an aqueous bottoms stream sent in part to the wash column and used as absorbent.

8. Process according to claims 5 and 2 or 6 and 2 wherein,
● the water stream recovered in the course of the compression of the contaminated hydrocarbon before entering the absorption zone,
● the remaining part of the aqueous phase recovered by cooling the stripping column overhead in the stripping zone and not used as a reflux, are sent to a flash drum to produce a gaseous overhead sent to the wash column to be washed and an aqueous bottoms stream sent in part to the wash column and used as absorbent,
the remaining part of said flash drum aqueous bottoms stream and the bottoms of the wash column are sent to a stripping column referred to as the water stripping column to produce an overhead stream comprising essentially oxygenated contaminants and hydrocarbons and an essentially pure water bottoms stream.

9. Process according to any one of the preceding claims wherein the hydrocarbon stream comprising oxygenated contaminants and water is the effluent produced by an alcohol dehydration to make at least an olefin.

10. Process according to any one of claims 1 to 8 wherein the hydrocarbon stream comprising oxygenated contaminants and water is the effluent produced by a MTO process.

11. Process according to any one of the preceding claims wherein the pressure of the absorption zone is ranging from 5 to 40 bars absolute.

12. Process according to any one of the preceding claims wherein the absorbent is selected from the group consisting of a polyol, amine, amide, nitrile, heterocyclic nitrogen containing compound, and mixtures thereof.

13. Process according to claim 12 wherein the absorbent is selected among ethylene glycol, diethylene glycol and triethylene glycol.
